# EUROPÄISCHE PATENTANMELDUNG

(11) **EP 3 735 960 A1**
(43) Veröffentlichungstag der Anmeldung: **11.11.2020**
(21) Anmeldenummer: 20172872.2
(22) Anmeldetag: 05.05.2020
(51) Int. Cl.: A61K 8/34, A61K 8/11, A61K 8/73, A61K 8/9789, A61K 8/49, A61K 8/64, A61Q 19/08, A61Q 19/00, A61Q 19/02

(54) **KOSMETISCHE ZUBEREITUNG ENTHALTEND RESVERATROL**

(30) Priorität: 06.05.2019 DE 102019111688
(71) Anmelder: BHI Beauty & Health Investment Group Management GmbH, 85774 Unterföhring (DE)
(72) Erfinder: Asam, Marcus, 81925 München (DE)
(74) Vertreter: btb IP Bungartz Baltzer Partnerschaft mbB Patentanwälte

(57) **Zusammenfassung**

Die Erfindung betrifft eine kosmetische Zubereitung, die eine Kombination aus in Mikrokapseln verkapseltem Resveratrol und unverkapseltem Resveratrol sowie vernetzte Hyaluronsäure und/oder ein Salz der Hyaluronsäure enthält. Die neue Wirkstoffkombination zeigt eine verbesserte Bioverfügbarkeit des Wirkstoffs Resveratrol mit allmählicher Wirkstoff-Freisetzung, wodurch das Risiko von Hautirritationen verringert und die Zellvitalität gesteigert werden und so Linien und Fältchen der Haut minimiert und die Hautzellen besser vor Alterungsprozessen geschützt werden.

## Beschreibung

Die vorliegende Erfindung betrifft eine kosmetische Zubereitung, die Resveratrol enthält. Ferner betrifft die Erfindung die Verwendung einer solchen kosmetischen Zubereitung als Mittel zum Auftragen auf die Haut zur Verringerung von Falten und zur Verbesserung des Hautbildes sowie die Verwendung einer Kombination von verkapseltem Resveratrol und unverkapseltem Resveratrol in kosmetischen Zubereitungen.

Die Alterungserscheinungen der Haut treten im Wesentlichen an der Grenze zwischen Epidermis und Dermis auf. Hinzukommt, dass ab einem Alter von 45 Jahren durch hormonelle Umstellungen sowohl die Epidermis als auch die Dermis allmählich dünner werden.

Der primäre Zelltyp in der Dermis sind die Fibroblasten. Ihre Hauptfunktion ist die Synthese und Erhaltung der extrazellulären Matrix (ECM), die Produktion von Collagen, Elastin, Proteoglicanen, Fibronectin und Glycosaminoglycanen. Diese Substanzen sind auch an Wundheilungsprozessen beteiligt, welche Schlüsselfaktoren für die Erhaltung einer gesunden Haut sind.

Bereits seit alters her versuchen die Menschen, die mit dem Alter einhergehenden biologischen Prozesse, welche auch die Haut verändern, zu beeinflussen und die sichtbaren Veränderungen der Haut, wie die Bildung von Falten, Verlust von Elastizität und Volumen der Haut zu stoppen bzw. zu verlangsamen. Üblicherweise werden Cremes, Lotionen und andere topisch zu applizierende Mittel verwendet, um diese Prozesse zu beeinflussen, das Erscheinungsbild der Haut zu verbessern.

Alle auf die Haut aufzutragenden Produkte enthalten Wirkstoffe, die das Hautbild verbessern sollen. Eine Wirkung kann erfahrungsgemäß nur dann erzielt werden, wenn die Wirkstoffe auch tatsächlich in den Hautzellen aktiv werden können, in denen die Alterungsprozesse stattfinden, nämlich in den Fibroblasten. Eine maximale Wirkung der einzelnen Inhaltsstoffe kann daher nur dann erzielt werden, wenn diese Wirkstoffe auch in die Fibroblasten transportiert werden können.

Aus der WO2016/135203 A1 ist der Einsatz der aktiven Komponenten in verkapselter der Form bekannt. Hier werden Mikrokapseln beschrieben, die mit Dodecapeptiden auf der Oberfläche funktionalisiert sind, wodurch sie, wenn sie auf die Haut aufgetragen werden, gezielt in die Fibroblasten transportiert werden.

Der Transport von Wirkstoffen in Richtung Fibroblasten ist eine wichtige Voraussetzung für die Verbesserung des Hautbildes, aber auch die richtige Kombination und Auswahl von Wirkstoffen ist wesentlich für die Wirkung, das heißt die Verbesserung des Hautbildes.

Der vorliegenden Erfindung lag die Aufgabe zugrunde, kosmetische Zubereitungen zur Verfügung zu stellen, die Wirkstoffe, welche der Haut ein jugendliches Aussehen verleihen, in einer geeigneten Kombination und Darreichungsform enthält, so dass bereits vorhandene Linien und Fältchen minimiert werden und die Haut ein pralles und glattes Erscheinungsbild zeigt.

Diese Aufgabewird nach der vorliegenden Erfindung durch eine kosmetische Zubereitung gelöst, die eine Kombination aus in Mikrokapseln verkapseltem Resveratrol und unverkapseltem Resveratrol sowie vernetzte Hyaluronsäure und/oder ein Salz der Hyaluronsäure enthält.

Die Wirkstoffkombination aus verkapseltem und unverkapseltem Resveratrol sowie vernetzter Hyaluronsäure zeigt eine verbesserte Bioverfügbarkeit des Wirkstoffs Resveratrol mit allmählicher Wirkstoff-Freisetzung. Durch die langsamere Wirkstoff-Freisetzung kann sich die Wirkung besser entfalten und das Risiko von Hautirritationen wird verringert. Die Erfinder haben festgestellt, dass durch die erfindungsgemäße Zubereitung die Zellvitalität gesteigert und auch die Collagensynthese aktiviert wird, wodurch das Aussehen der Haut optimiert wird. Die vernetzte Hyaluronsäure bindet gleichzeitig Feuchtigkeit und unterstützt auf diese Weise ein glattes Erscheinungsbild der Haut.

Resveratrol ist ein Polyphenol, das natürlicherweise in Pflanzen enthalten ist, vor allem in Weintrauben, Weinreben, Himbeeren, Maulbeeren, Pflaumen, Erdnüssen und im japanischen Staudenknöterich. Es kommt als trans- und cis-lsomer in monomerer und oligomerer Form vor und kann aus den Pflanzen isoliert, mit biotechnologischen und auch synthetischen Verfahren hergestellt werden. Das aus Pflanzen isolierte Produkt ist im Handel in der Regel in verdünnter Form erhältlich. In der erfindungsgemäßen Zubereitung wird Resveratrol vorzugsweise als Bestandteil eines Pflanzenextrakts eingesetzt, wobei Weinrebenextrakt oder Traubenkernextrakt besonders bevorzugt sind. In den Resveratrol enthaltenden Mikrokapseln wird vorzugsweise Traubenkernextrakt eingesetzt. Das unverkapselte Resveratrol ist vorzugsweise ein aus Weinreben gewonnener Pflanzenextrakt oder ein mit biotechnologischen Verfahren hergestelltes Resveratrol. Es können auch Gemische von Resveratrol aus unterschiedlichen Herstellungsverfahren eingesetzt werden. Resveratrol ist im Handel erhältlich, beispielsweise unter den Handelsbezeichnungen Veri-te™ Resveratrol (Evolva) oder Resveratrox™ (Actichem). Die Resveratrol enthaltenden Mikrokapseln können in der erfindungsgemäßen Zubereitung in einer Menge von 0,000005 bis 0,005 Gew.-%, bezogen auf die kosmetische Zubereitung, enthalten sein.

Weinrebenextrakt in unverkapselter Form ist vorzugsweise in einer Menge von 0,0001 bis 05 Gew.-%, bezogen auf die kosmetische Zubereitung, enthalten.

Als weiteren Inhaltsstoff enthält die erfindungsgemäße kosmetische Zubereitung vernetzte Hyaluronsäure oder ein für eine kosmetische Anwendung geeignetes Salz oder ein Gemisch aus den voranstehenden. Als geeignete Salze kommen insbesondere die Alkalisalze in Betracht, bevorzugt das Natriumsalz. Die vernetzte Hyaluronsäure bindet Feuchtigkeit, wodurch die Haut ein glattes Erscheinungsbild erhält. Vernetzte Hyaluronsäure ist im Handel erhältlich und kann erhalten werden aus Hyaluronsäure oder einem Derivat, wie einem Polyaldehyd, mit einem geeigneten Vernetzungsmittel, beispielsweise einem Diamin oder Bisoxyamin. Die vernetzte Hyaluronsäure kann in der vernetzten Struktur auch freie Hyaluronsäure oder Hyaluronsäuresalze enthalten. Die vernetzte Hyaluronsäure kann in der erfindungsgemäßen kosmetischen Zubereitung in einer Menge von 0,00005 Gew.-% bis 1 Gew.-%, insbesondere zwischen 0,00005 Gew.-% bis 0,5 Gew.-%, bezogen auf die kosmetische Zubereitung, enthalten sein, wobei die vernetzte Hyaluronsäure in pflegenden und reinigenden Produkten, wie Cremes etc., in größeren Mengen eingesetzt wird als in reinigenden Produkten, wie in einem Duschgel.

Die erfindungsgemäße Zubereitung kann neben Resveratrol noch weitere Wirkstoffe enthalten, wie oligomere Proanthocyanidine (OPC). Diese oligomeren Proanthocyanidine sind beispielsweise in Traubenkernextrakt enthalten. In einer möglichen Ausführungsform der vorliegenden Erfindung ist in den Mikrokapseln OPC-haltiger Traubenkernextrakt enthalten.

Erfindungsgemäß enthält die kosmetische Zubereitung in Mikrokapseln verkapseltes Resveratrol und ggf. weitere Inhaltsstoffe. Die Materialien, aus welchem die Mikrokapseln hergestellt sind, können übliche aus dem Stand der Technik für die Mikroverkapselung bekannte Polymere sein. Vorzugsweise sind die Polymere ausgewählt ist aus der Gruppe bestehend aus Poly(D,L-lactide-co-glycolid), Polymilchsäure, Poly(propylenfumarat-co-ethyleneglycol)-Blockcopolymer [P(PF-co-EG)], Polyanhydrid, Poly(fumarsäure-co-sebacinsäure)anhydrid, Poly(ethyleneoxid)-poly(lactid/glycolid), Polyvinylalkohol, Alginat, Dextran, Chitosan, Hydroxyapatit, Collagen, Fibrin, Hyaluronsäure, Carbomeren, Polyaminosäuren and Polyethylenglylol und Copolymeren sowie Derivaten der voranstehenden.

In einer bevorzugten Ausführungsform weist die Kapselwand der erfindungsgemäß verwendeten Mikrokapseln eine Doppelschicht auf, das bedeutet, die Kapselwand hat eine innere Polymerschicht und eine äußere Polymerschicht, die die äußere Oberfläche der Kapsel bildet. Beide Schichten werden vorzugsweise aus den oben genannten Polymeren gebildet. In einer besonders bevorzugten Ausführungsform werden die innere Wandung und die äußere Wandung von unterschiedlichen Polymeren gebildet. Besonders bevorzugt ist die innere Kapselwand aus Poly(D,L-lactid-co-glycolid) (PLGA) und die äußere Kapselwand aus Polyvinylalkohol (PVA). Vorzugsweise sollte die Doppelwandung der Mikrokapseln so gestaltet sein, dass sich einige der funktionellen Gruppen des Polymers der inneren Schicht auf der äußeren Oberfläche der Kapsel befinden. In dieser Ausführungsform liegt das molare Verhältnis Lactid/Glycolid im PLGA zwischen 40:60 bis 60:40, insbesondere bei etwa 50:50.

In einer weiteren bevorzugten Ausführungsform weist die äußere Oberfläche der Mikrokapseln Oligopeptide mit 6 bis 18 Aminosäuren auf, die kovalent an das Polymer der Kapselwand gebunden sind. Besonders bevorzugt ist diese kovalente Bindung eine Amidbindung zwischen der Amino-Gruppe der N-terminalen Gruppe des Peptids und der Carboxyl-Gruppe des PLGA-Polymers, die sich an der Oberfläche der Mikrokapseln befindet.

Die Oligopeptide sind sogenannte Steuerpeptide, die eine hohe Affinität zum Fibroblasten-Rezeptor (FGF) aufweisen. Es wurde festgestellt, dass Mikrokapseln, die an ihrer Oberfläche ein Steuerpeptid aufweisen, gezielt an Fibroblasten andocken und die Wirkstoffabgabe direkt an bzw. in die Fibroblasten, also direkt am Wirkort, erfolgt. Der gezielte Transport an den Wirkort hat den Vorteil, dass die Menge an Wirkstoff verringert werden kann mit der Folge, dass auch die Gefahr von potentiellen Nebenwirkungen reduziert wird. Ein weiterer Vorteil dieser Mikrokapseln ist, dass sie in die Haut eindringen können und sich gleichmäßig über die gesamte Epidermis verteilen, was auch zu einer gleichmäßigen Verteilung des Wirkstoffs führt.

Die bevorzugt eingesetzten Oligopeptide enthalten üblicherweise die aus dem Stand der Technik bekannten Aminosäuren, nämlich Alanin, Phenylalanin, Lysin, Glutamin und/oder Arginin. Die Herstellung der erfindungsgemäßen Mikrokapseln kann nach aus dem Stand der Technik bekannten Verfahren erfolgen. Mikrokapseln, die auf der äußeren Oberfläche Oligopeptide aufweisen, können ebenfalls mit Verfahren erhalten werden, die dem Fachmann bekannt sind, beispielsweise nach dem Verfahren, wie es in der WO2016/135203 A1 beschrieben ist.

Die erfindungsgemäße Zubereitung wird insbesondere als Pflegeprodukt oder als Reinigungsprodukt auf die Haut aufgetragen. Vorzugsweise liegt diese Zubereitung in Form von Emulsionen, Salben, Gelen, Ölen, Wachsen, Seifen, Balsamen, Seren oder tensidischen Systemen vor. Diese Zubereitungen können zu beliebigen, in der Hautpflege üblichen Produktformen verarbeitet und im Handel angeboten werden, z. B. in Form einer Creme, einer Lotion, einer Maske, einer Milch, eines Schaums, eines Aerosols, eines Sticks, eines Patches, eines Duschgels, einer Reinigungscreme, einer Ampulle, einer Tuchmaske usw.

Die kosmetische Zubereitung kann als weitere Inhaltsstoffe solche Substanzen enthalten, die üblicherweise in kosmetischen Zubereitungen enthalten sind, beispielsweise Wasser, Öle, Wachse, Feuchthaltemittel, Emulgatoren, Fettalkohole, Verdickungsmittel und Duftstoffe, Konservierungsmittel, Pigmente, Komplexbildner, Antioxidantien, antimikrobiell wirkende Substanzen, pH-Stellmittel, UV-Filter, Vitamine, Provitamine, neben Resveratrol weitere Pflanzenextrakte usw. Sofern die kosmetische Zubereitung ein Mittel zur Reinigung der Haut ist, können darüber hinaus Tenside, rückfettende Mittel und feuchthaltende Mittel enthalten sein. Je nach Anwendungszweck kann die erfindungsgemäße Zubereitung auch sogenannte mineralische oder pflanzliche Peeling-Körper enthalten.

Die neue kosmetische Zusammensetzung wird vorzugsweise zur Pflege und Behandlung der Haut verwendet, insbesondere zur Vorbeugung und Behandlung von Alterungsprozessen der Haut und zur Verringerung von Faltenbildung sowie der Verbesserung der Strahlkraft und der Elastizität der Haut, Verringerung der Hautrauigkeit sowie Erhöhung der Hautdicke und Verringerung von altersbedingten Pigmentstörungen.

### Beispiele

### Gesichtscreme

| **Komponente** | | **Anteil in [%]** |
|---|---|---|
| Öle/Emollients | | 11 |
| Wachse/Buttern | | 2 |
| Feuchhaltemittel | | 6,5 |
| Emulgatoren | | 1,8 |
| Fettalkohole | | 3,5 |
| Verdickungsmittel/Konsistenzgeber | | 1 |
| Wirkstoff | | 4,8401 |

| die Wirkstoffmenge enthält (bezogen auf die fertige Zusammensetzung) | | |
|---|---|---|
| • vernetzte Hyaluronsäure | 0,01 | |
| • unverkapseltes Resveratrol aus Weinreben extra kt | 0,2 | |
| • biotechnologisch hergestelltes Resveratrol | 0,2 | |
| • verkapseltes Resveratrol mit OPC | 0,0001 | |
| Parfum | | 0,4 |
| Sonstige Stoffe | | 2,17 |
| Wasser | | ad 100 |

### Augencreme

| **Komponente** | | **Anteil in [%]** |
|---|---|---|
| Öle/Emollients | | 14,1 |
| Wachse/Buttern | | 3 |
| Feuchhaltemittel | | 14 |
| Emulgatoren | | 5,4 |
| Fettalkohole | | 4,2 |
| Verdickungsmittel/Konsistenzgeber | | 0,6 |
| Wirkstoff | | 4,5802 |

| der Wirkstoff enthält (bezogen auf die fertige Zusammensetzung) | | |
|---|---|---|
| • vernetzte Hyaluronsäure | 0,1 | |
| • unverkapseltes Resveratrol aus Weinreben extra kt | 0,40 | |
| • biotechnologisch hergestelltes Resveratrol | 0,0001 | |
| • verkapseltes Resveratrol mit OPC | 0,0001 | |
| Parfum | | 0 |
| Sonstige Stoffe | | 4,36 |
| Wasser | | 49,7598 |

### Serum

| **Komponente** | | **Anteil in [%]** |
|---|---|---|
| Öle/Emollients | | 12,2 |
| Wachse/Buttern | | 0 |
| Feuchhaltemittel | | 6,75 |
| Emulgatoren | | 4 |
| Fettalkohole | | 1 |
| Verdickungsmittel/Konsistenzgeber | | 0,6 |
| Wirkstoff | | 3,6411 |

| der Wirkstoff enthält (bezogen auf die fertige Zusammensetzung) | | |
|---|---|---|
| • vernetzte Hyaluronsäure | 0,01 | |
| • unverkapseltes Resveratrol aus Weinrebenextrakt | 1,0 | |
| • biotechnologisch hergestelltes Resveratrol | 0,0001 | |
| • verkapseltes Resveratrol mit OPC | 0,001 | |
| Parfum | | 1,1 |
| Sonstige Stoffe | | 1,65 |
| Wasser | | 70,0089 |

### Ampullenkur

| **Komponente** | | **Anteil in [%]** |
|---|---|---|
| Öle/Emollients | | 0,1 |
| Wachse/Buttern | | 0 |
| Feuchhaltemittel | | 16,5 |
| Emulgatoren | | 1 |
| Fettalkohole | | 0 |
| Verdickungsmittel/Konsistenzgeber | | 0,3 |
| Wirkstoff | | 7,6021 |

| der Wirkstoff enthält (bezogen auf die fertige Zusammensetzung) | | |
|---|---|---|
| • vernetzte Hyaluronsäure | 0,001 | |
| • unverkapseltes Resveratrol aus Weinreben extra kt | 1,0 | |
| • biotechnologisch hergestelltes Resveratrol | 0,001 | |
| • verkapseltes Resveratrol mit OPC | 0,0001 | |
| Parfum | | 0 |
| Sonstige Stoffe | | 1,07 |
| Wasser | | ad 100 |

### Maske

| **Komponente** | | **Anteil in [%]** |
|---|---|---|
| Öle/Emollients | | 10,3 |
| Wachse/Buttern | | 1,85 |
| Feuchhaltemittel | | 4 |
| Emulgatoren | | 5,8 |
| Fettalkohole | | 3,8 |
| Verdickungsmittel/Konsistenzgeber | | 3,5 |
| Wirkstoff | | 6,0312 |

| der Wirkstoff enthält (bezogen auf die fertige Zusammensetzung) | | |
|---|---|---|
| • vernetzte Hyaluronsäure | 0,001 | |
| • unverkapseltes Resveratrol aus Weinrebenextrakt | 3,0 | |
| • biotechnologisch hergestelltes Resveratrol | 0,0001 | |
| • verkapseltes Resveratrol mit OPC | 0,0001 | |
| Parfum | | 1,6 |
| Sonstige Stoffe | | 4,15 |
| Wasser | | ad 100 |

### Körpercreme

| **Komponente** | | **Anteil in [%]** |
|---|---|---|
| Öle/Emollients | | 8,8 |
| Wachse/Buttern | | 17,5 |
| Feuchhaltemittel | | 5,5 |
| Emulgatoren | | 2,75 |
| Fettalkohole | | 3 |
| Verdickungsmittel/Konsistenzgeber | | 0,9 |
| Wirkstoff | | 1,4503 |

| der Wirkstoff enthält (bezogen auf die fertige Zusammensetzung) | | |
|---|---|---|
| • vernetzte Hyaluronsäure | 0,0001 | |
| • unverkapseltes Resveratrol aus Weinreben extra kt | 0,5 | |
| • biotechnologisch hergestelltes Resveratrol | 0,0001 | |
| • verkapseltes Resveratrol mit OPC | 0,0001 | |
| Parfum | | 1,8 |
| Sonstige Stoffe | | 1,15 |
| Wasser | | ad 100 |

### Handcreme

| **Komponente** | | **Anteil in [%]** |
|---|---|---|
| Öle/Emollients | | 11 |
| Wachse/Buttern | | 3 |
| Feuchhaltemittel | | 3 |
| Emulgatoren | | 13,7 |
| Fettalkohole | | 0,5 |
| Verdickungsmittel/Konsistenzgeber | | 0,4 |
| Wirkstoff | | 8,4511 |

| der Wirkstoff enthält (bezogen auf die fertige Zusammensetzung) | | |
|---|---|---|
| • vernetzte Hyaluronsäure | 0,001 | |
| • unverkapseltes Resveratrol aus Weinreben extra kt | 0,4 | |
| • biotechnologisch hergestelltes Resveratrol | 0,0001 | |
| • verkapseltes Resveratrol mit OPC | 0,0001 | |
| Parfum | | 1,6 |
| Sonstige Stoffe | | 1,7 |
| Wasser | | ad 100 |

### Reinigungscreme

| **Komponente** | | **Anteil in [%]** |
|---|---|---|
| Öle/Emollients | | 15 |
| Wachse/Buttern | | 0 |
| Feuchhaltemittel | | 5 |
| Emulgatoren | | 7,6 |
| Fettalkohole | | 4 |
| Verdickungsmittel/Konsistenzgeber | | 0,45 |
| Wirkstoff | | 2,6503 |
| der Wirkstoff enthält (bezogen auf die fertige Zusammensetzung) | | |
| • vernetzte Hyaluronsäure | 0,0001 | |
| • unverkapseltes Resveratrol aus Weinrebenextrakt | 0,1 | |
| • biotechnologisch hergestelltes Resveratrol | 0,0001 | |
| • verkapseltes Resveratrol mit OPC | 0,0001 | |
| Parfum | | 1,6 |
| Sonstige Stoffe | | 1,3 |
| Wasser | | ad 100 |

### Duschgel

| **Komponente** | | **Anteil in [%]** |
|---|---|---|
| Öle/Emollients | | 0 |
| Wachse/Buttern | | 0 |
| Feuchhaltemittel | | 5 |
| Emulgatoren | | 37,5 |
| Fettalkohole | | 0 |
| Verdickungsmittel/Konsistenzgeber | | 0 |
| Wirkstoff | | 0,00021 |

| der Wirkstoff enthält (bezogen auf die fertige Zusammensetzung) | | |
|---|---|---|
| • vernetzte Hyaluronsäure | 0,0001 | |
| • unverkapseltes Resveratrol aus Weinreben extra kt | 0,0001 | |
| • biotechnologisch hergestelltes Resveratrol | 0 | |
| • verkapseltes Resveratrol mit OPC | 0,00001 | |
| Parfum | | 1,1 |
| Sonstige Stoffe | | 4,01 |
| Wasser | | ad 100 |

### Bodylotion

| **Komponente** | | **Anteil in [%]** |
|---|---|---|
| Öle/Emollients | | 10 |
| Wachse/Buttern | | 3,5 |
| Feuchhaltemittel | | 6 |
| Emulgatoren | | 2 |
| Fettalkohole | | 2 |
| Verdickungsmittel/Konsistenzgeber | | 0,7 |
| Wirkstoff | | 0,7303 |

| der Wirkstoff enthält (bezogen auf die fertige Zusammensetzung) | | |
|---|---|---|
| • vernetzte Hyaluronsäure | 0,0001 | |
| • unverkapseltes Resveratrol aus Weinreben extra kt | 0,2 | |
| • biotechnologisch hergestelltes Resveratrol | 0,0001 | |
| • verkapseltes Resveratrol mit OPC | 0,0001 | |
| Parfum | | 1,0 |
| Sonstige Stoffe | | 2,70 |
| Wasser | | ad 100 |

### Peeling

| **Komponente** | | **Anteil in [%]** |
|---|---|---|
| Öle/Emollients | | 4,9 |
| Wachse/Buttern | | 3,3 |
| Feuchhaltemittel | | 2,5 |
| Emulgatoren | | 2,85 |
| Fettalkohole | | 2,5 |
| Verdickungsmittel/Konsistenzgeber | | 1,3 |
| Wirkstoff | | 0,5503 |
| der Wirkstoff enthält (bezogen auf die fertige Zusammensetzung) | | |
| • vernetzte Hyaluronsäure | 0,0001 | |
| • unverkapseltes Resveratrol aus Weinreben extra kt | 0,02 | |
| • biotechnologisch hergestelltes Resveratrol | 0,0001 | |
| • verkapseltes Resveratrol mit OPC | 0,0001 | |
| Parfum | | 0,8 |
| Sonstige Stoffe | | 6,1 |
| Wasser | | ad 100 |

### Getönte Tagescreme

| **Komponente** | | **Anteil in [%]** |
|---|---|---|
| Öle/Emollients | | 22 |
| Wachse/Buttern | | 0 |
| Feuchhaltemittel | | 6 |
| Emulgatoren | | 8,5 |
| Fettalkohole | | 0 |
| Verdickungsmittel/Konsistenzgeber | | 0,5 |
| Wirkstoff | | 1,1142 |
| der Wirkstoff enthält (bezogen auf die fertige Zusammensetzung) | | |
| • vernetzte Hyaluronsäure | 0,001 | |
| • unverkapseltes Resveratrol aus Weinrebenextrakt | 0,05 | |
| • biotechnologisch hergestelltes Resveratrol | 0,0001 | |
| • verkapseltes Resveratrol mit OPC | 0,0001 | |
| Parfum | | 1,6 |
| Sonstige Stoffe | | 12,1 |
| Wasser | | ad 100 |

## Patentansprüche

1. Kosmetische Zubereitung enthaltend eine Kombination aus in Mikrokapseln verkapseltem Resveratrol und unverkapseltem Resveratrol sowie vernetzte Hyaluronsäure und/oder ein Salz der Hyaluronsäure.

2. Kosmetische Zubereitung nach Anspruch 1, **dadurch gekennzeichnet, dass** das Resveratrol als Bestandteil von Weinrebenextrakt enthalten ist.

3. Kosmetische Zubereitung nach einem der Ansprüche 1 oder 2, **dadurch gekennzeichnet, dass** das unverkapselte Resveratrol als Bestandteil von Weinrebenextrakt oder als aus biotechnologischen oder synthetischen Verfahren erhaltenes Resveratrol oder als Gemisch der voranstehenden enthalten ist.

4. Kosmetische Zubereitung nach einem der Ansprüche 1 bis 3, **dadurch gekennzeichnet, dass** die Mikrokapseln neben Resveratrol Traubenkernextrakt enthalten.

5. Kosmetische Zubereitung nach einem der Ansprüche 1 bis 4, **dadurch gekennzeichnet, dass** die vernetzte Hyaluronsäure in einer Menge von 0,00005 Gew.-% bis 1 Gew.-%, bezogen auf die Zubereitung, enthalten ist.

6. Kosmetische Zubereitung nach einem der Ansprüche 1 bis 5, **dadurch gekennzeichnet, dass** oligomere Proanthocyanidine (OPC) enthalten sind.

7. Kosmetische Zubereitung nach einem der Ansprüche 1 bis 6, **dadurch gekennzeichnet, dass** das Material der Mikrokapseln ausgewählt ist aus der Gruppe bestehend aus Poly(D,L-lactide-co-glycolide), Polylmilchsäuren, Poly(propylenfumarat-co-ethyleneglycol)-Blockcopolymeren [P(PF-co-EG)], Polyanhydride poly(fumarsäure-co-sebacinsäure)anhydrid, Poly(ethyleneoxid)-poly(lactid/glycolid), Polyvinylalkohol, Alginaten, Dextran, Chitosan, Hydroxyapatit, Collagen, Fibrin, Hyaluronsäure, Carbomeren, Polyaminosäuren and Polyethylenglycol.

8. Kosmetische Zubereitung nach einem der Ansprüche 1 bis 7, **dadurch gekennzeichnet, dass** die Mikrokapseln ausgewählt sind aus doppelwandigen Mikrokapseln auf deren Oberfläche ein Oligopeptid mit 6 bis 18 Aminosäuren gebunden ist.

9. Kosmetische Zubereitung nach einem der Ansprüche 1 bis 8, **dadurch gekennzeichnet, dass** die kosmetische Zubereitung eine Emulsion, eine Salbe, ein Gel, ein Öl, ein Wachs, ein Balsam, ein Serum oder ein tensidisches System ist.

10. Verwendung einer kosmetischen Zubereitung nach einem der Ansprüche 1 bis 9 zum topischen Auftragen auf die Haut zur Verringerung von Hautfalten und/oder zur Verbesserung des Hautbildes.
